# EUROPEAN PATENT SPECIFICATION

(11) **EP 2 399 123 B1**
(45) Date of publication and mention of the grant of the patent: **09.09.2020**
(21) Application number: 10705205.2
(22) Date of filing: 19.02.2010
(51) Int. Cl.: G01N 30/86

(54) **A METHOD, A SYSTEM AND A COMPUTER PROGRAM FOR A SIMULTANEOUS DATA PROCESSING FOR AN AUTOMATIC EXTRACTION OF RESPECTIVE PEAKS IN MULTIPLE CHROMATOGRAPHIC SPECTRA**
VERFAHREN, SYSTEM UND COMPUTERPROGRAMM ZUR DATENVERARBEITUNG FÜR EINE AUTOMATISCHE EXTRAKTION JEWEILIGER PEAKS IN EINEM CHROMATOGRAPHISCHEN SPEKTRUM
Procédé, système et programme informatique pour le traitement de données pour une extraction automatique des pics correspondants dans un spectre chromatographique

(30) Priority: 20.02.2009 EP 09153346
(43) Date of publication of application: 28.12.2011
(73) Proprietor: Nederlandse Organisatie voor toegepast- natuurwetenschappelijk onderzoek TNO, 2595 DA 's-Gravenhage (NL)
(72) Inventor: JELLEMA, Renger Henk, 3532 TL Utrecht (NL); VOGELS, Jacobus Thomas Wilhelmus Elisabeth, 3700 AJ Zeist (NL); VAN DER KLOET, Frans Meindert, 2628 VK Delft (NL); THISSEN, Uwe Maria Johannes, 2628 VK Delft (NL)
(74) Representative: V.O.
(86) International application number: PCT/NL2010/050083
(87) International publication number: WO 2010/095941

(56) References cited:
- WO-A-2006/110848
- WO-A-2007/012643
- WO-A1-2004/038602
- WO-A2-2005/079263
- DE-A1- 10 051 806
- GB-A- 2 410 608
- US-A1- 2005 273 276
- US-A1- 2006 271 310
- US-A1- 2007 023 633
- KONG H ET AL: "Deconvolution of overlapped peaks based on the exponentially modified Gaussian model in comprehensive two-dimensional gas chromatography" JOURNAL OF CHROMATOGRAPHY, ELSEVIER SCIENCE PUBLISHERS B.V. AMSTERDAM, NL, vol. 1086, no. 1-2, 9 September 2005 (2005-09-09), pages 160-164, XP004995151 ISSN: 0021-9673
- ZHANG Z ET AL: "A Universal Algorithm for Fast and Automated Charge State Deconvolution of Electrospray Mass-to-Charge Ratio Spectra" JOURNAL OF THE AMERICAN SOCIETY FOR MASS SPECTROMETRY, ELSEVIER SCIENCE INC, US, vol. 9, no. 3, 1 March 1998 (1998-03-01), pages 225-233, XP004112012 ISSN: 1044-0305
- W Gary Mallard ET AL: "Automated Mass Spectral Deconvolution & Identification System AMDIS - USER GUIDE", , 1 January 1997 (1997-01-01), XP055390075, Retrieved from the Internet: URL:http://chemdata.nist.gov/mass-spc/amdi s/docs/amdis.pdf [retrieved on 2017-07-12]

## Description

### FIELD

The invention relates to a method of data processing for automatic extraction of respective peaks in multiple chromatographic spectra simultaneously. The invention further relates to a system for data processing for automatic extraction of respective peaks in multiple chromatographic spectrum simultaneously. The invention still further relates to a computer program for data processing for automatic extraction of respective peaks in multiple chromatographic spectra simultaneously.

### BACKGROUND

An embodiment of a method as is set forth in the opening paragraph is known from US 2005/0273276. In the known method a chromatographic spectrum comprising a plurality of convoluted peaks is processed using four dedicated software packages. First software package is arranged for an initial analysis of the chromatographic spectra using retention time analysis and mass spectra analysis. As a first output deconvoluted peaks are generated. The second software package is arranged to receive results of the first software package for generating a second result further identifying and/or confirming the identity of the target compounds of the first output. The third software relates to the NIST02 library software package and is arranged to receive the second result for generating a third result still further confirming the identity of the target compounds. Finally, the fourth software package is arranged to receive the first, the second and the third results for confirming the identity of the target compound by sorting the first, second and the third results according to predefined criteria. For example, retention time, four ion analysis and chemical abstracts service number may be used for such predefined criteria.

GB 2 410 608 A discloses a method for investigating chromatographic mass spectra for patterns known from a library, and a confidence level value indicative of a measure of the fit between the spectrum and the library of known spectra is assigned.

US 2007/023633 A1 relates to analysis of chromatography / mass spectrometry data by deconvolution of the mass spectral data.

WO 2005/079263 A2 relates to an apparatus and a method for identifying chromatographic peaks, the method comprising a step of choosing a retention time window and then interrogating a parameter table.

It is a disadvantage of the known method that a complex, multi-step identification procedure has to be followed for deconvoluting and identifying individual spectra in the total ion chromatogram.

Simultaneous deconvolution of multiple spectra is understood to relate to identification of compounds found in multiple spectra, by automatically tracking and identifying small differences in retention times between the individual spectra. Simultaneous deconvolution of multiple spectra puts a substantial demand on both computer memory and processor time. Processing a spectrum estimated to contain 4 peaks takes about 4 times as long to process as a block estimated to contain 2 peaks. Processing a spectrum containing more than 100 peaks will take hours. This has to be repeated for multiple files.

### SUMMARY OF THE INVENTION

It is an object of the invention to provide a method for automatic simultaneous deconvolution of individual peaks in multiple chromatograms, which is simple, yet robust and reliable.

To this end the method of simultaneous data processing for an automatic extraction of respective peaks in multiple chromatographic spectra according to claim 1 is provided.

According to the invention, in order to limit both computational demands the spectra are first combined into a single block of data which are then split out again into smaller individual blocks i.e. strips of peaks in a small retention time window. A Block is constructed over all masses in all spectra with a width of typically 4 times the estimated peakwidth of an average single peak. Processing 5 spectra with each 100 mass traces and an average peakwidth of 20 points will typically result in a block of (5 ^{∗} 100) by (4^{∗}20) points. Both the average peakwidth and the width factor of 4 can be adapted depending on the data. Typically processing GCMS - spectra will use a factor of 4 while LCMS can use larger factors. Each block is then deconvoluted resulting in a set of block results containing a set of spectra and chromatograms for each block separately. An Alternating Least Squares method may be used for deconvolution. As the used Alternating Least Squares method may be less reliable for peaks along the edges of the retention time blocks. Only peaks extracted from the central 50% of the block size are reliable. The blocks are therefore defined so that they partly overlap with the previous and the next block. For example, a block may overlap with 50% with the previous block and by 50% with the following block along the retention time scale. This will result in a global doubling of peaks. The method of the invention may comprise the step of selecting only unique one set of peaks from overlapping blocks that are located closed to the centre of their respective blocks. This will result in a grand overall list of reliable spectra and chromatograms for the whole collection of samples.

A challenging step in this process is the estimation of the number of compounds in a block. Usual rank estimation to estimate the number of peaks may not be satisfactory as small changes in the retention time of the spectra increase the estimation of the number of peaks in the block. A small RT change may be indistinguishable from noise on the peaks thus degenerating the rank structure of the block.

To solve this problem in the method according to the invention a stepwise extraction of compounds from the block structure is used, followed by evaluation of each spectrum and chromatogram as it is extracted. The extraction is stopped when either the spectral quality is low or when no match in the reference compound database is found When the latter occurs the block is labelled and flagged for an optional future manual deconvolution.

It is found that using prior knowledge, for example knowledge on expected constituents in the chromatographic spectrum under consideration, substantial computation time may be saved without compromising deconvolution accuracy. In particular, according to the method of the invention, the characteristic peaks are used for fitting the integral chromatographic spectrum using multivariate statistical analysis. It will be appreciated that methods of the multivariate statistical analysis are known per se and application thereof to chromatographic spectra falls within ordinary skill of the person skilled in the art.

It is found to be advantageous to fit the chromatographic spectra using multivariate statistical analysis as in course thereof respective characteristic spectra or portions thereof may be suitably shifted along a fitting axis, for example, along retention time axis. As a result, the method according to the invention may be more flexible and, thus, more successful in deconvoluting distorted spectra, as no perfect match between the characteristic spectra from the database and a best fit for the chromatographic spectrum may be required. The method known from US 2005/0273276 may provide inferior results, as it operates using some mathematical processes resolving unresolved compounds based on standard library data, wherein characteristic peaks may be treated as rigid entities having pre-determined and non alterable positions along a fitting axis.

In an embodiment of the method according to the invention, the method comprises the step of assigning a confidence level to the extracted peaks in the chromatogram.

It is found that for some chromatographic spectra particular peaks may not be fully or adequately resolved. For example, when the sample to be analyzed comprises unexpected peaks, or when the background level is too high for some areas. It is, therefore, found to be particularly advantageous to provide the method according to the invention with an internal quality check, by allowing an automatic assignment of a confidentiality level to the extracted peaks. In this way, when a peak with a confidentiality level below a pre-determined threshold is signalled, an automatic or manual re-fit may be carried out.

Preferably, the method according to the invention generates an automatic report based on the result of deconvolution of the chromatographic spectrum. It is found to be advantageous to supplement peaks identified in the report with their respective confidence level. However, it may be also possible that only peaks with an unacceptable confidence level are marked, for example peaks having a confidence level below a pre-determined level may be underlined, labelled or highlighted in any other suitable way.

In a further embodiment of the method according to the invention, wherein the chromatogram comprises a residual signal post extraction of the respective peaks, the method according to the invention further comprises the step of extracting residual peaks from the chromatogram based on a residual signal.

It is found to be advantageous for extraction of peaks corresponding to trace elements to first deconvolute major peaks including their intrinsic subpeaks, followed by deconvolution of residual peaks. In this way accuracy for identification of residual peaks may be increased.

A computer program product according to claim 7 comprises instructions for causing a processor to carry out the steps of the method as is set forth with reference to the foregoing.

Furthermore, a system according to claim 8 is provided.

It will be appreciated that the system according to the invention may be operable at a different location with respect to a physical location of the databases of characteristic peaks. For example, the database may be provided on a specific server which may be accessible by different users, like different laboratories, hospitals or the like. Preferably, the system according to the invention comprises the database which may lead to an increased efficiency of the system as data transport is carried out locally. More preferably, the system according to the invention comprises a chromatography unit. In this case data analysis may be performed substantially instantly after generating the chromatographic spectra, which may be advantageous, especially when it is desired to repeat the step of chromatography on the same or on a similar sample for improving deconvolution accuracy.

These and other aspects of the invention will be discussed in more detail with reference to drawings, wherein like reference numerals refer to like elements. It will be appreciated that the drawings are presents for illustrative purposes and may not be used for limiting the scope of the appended claims.

### BRIEF DESCRIPTION OF THE DRAWINGS

Figure 1 depicts in a schematic way a method to construct blocks of data.
Figure 2 depicts in a schematic way an embodiment of a chromatographic spectrum comprising three convoluted peaks;
Figure 3 depicts in a schematic way an embodiment of resolved peaks substantially constituting the chromatogram of Figure 1;
Figure 4 depicts in a schematic way convoluted spectra for peaks 2 and 3 of Figure 1;
Figure 5 depicts in a schematic way characteristic peaks for peak 1 of Figure 1;
Figure 6 depicts in a schematic way characteristic peaks for peak 2 of Figure 1;
Figure 7 depicts in a schematic way characteristic peaks for peak 3 of Figure 1;
Figure 8 depicts in a schematic way an embodiment of a flow-chart of a computer program product according to the invention;
Figure 9 depicts in a schematic way an embodiment of a system according to the invention.

### DETAILED DESCRIPTION OF THE DRAWINGS

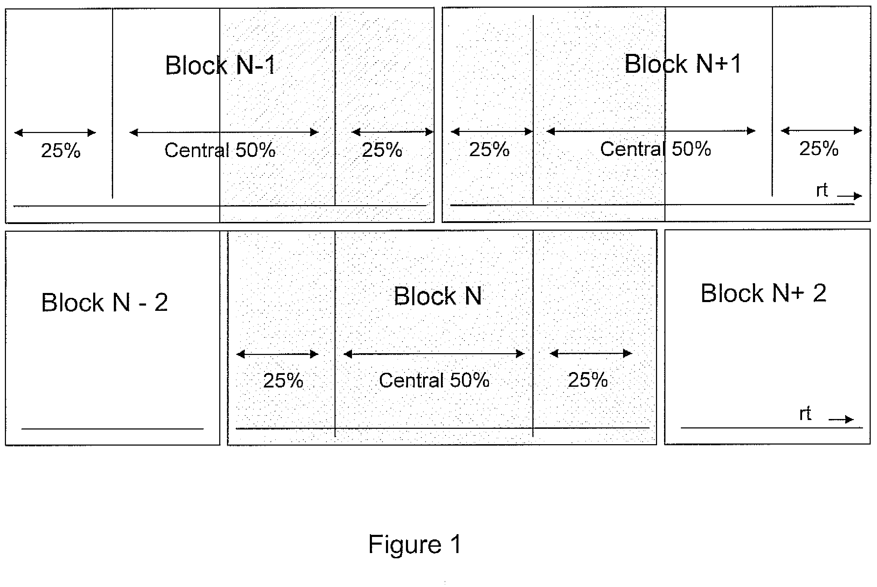

Figure 1 depicts in a schematic way an embodiment of a the global data into a set of overlapping blocks. 50% of block N will overlap with 50% of block N-1 and the other 50% of the block will overlap with 50% of the next block. After deconvolution peaks (spectra and chromatograms) found in the outer 25% (left or right) of the central region will less reliable than those found in the central 50% of the block.

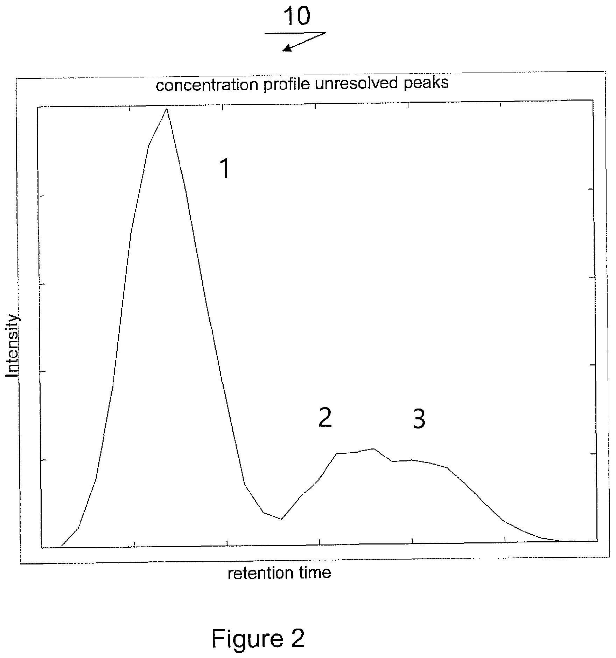

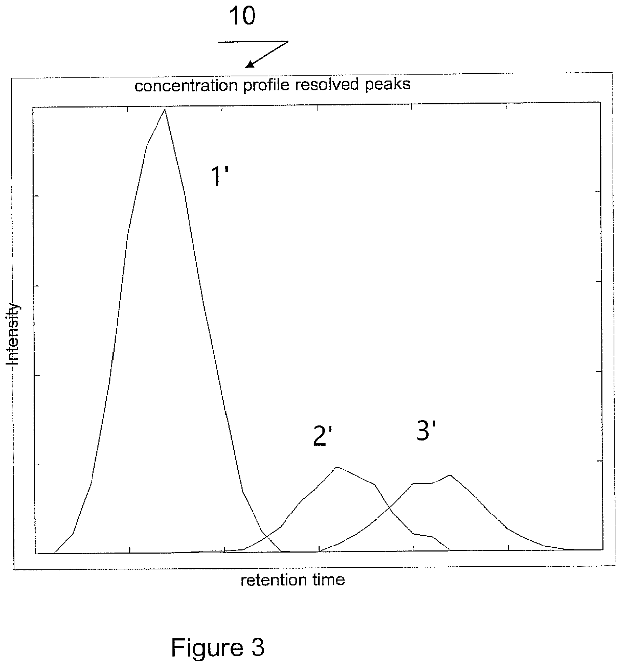

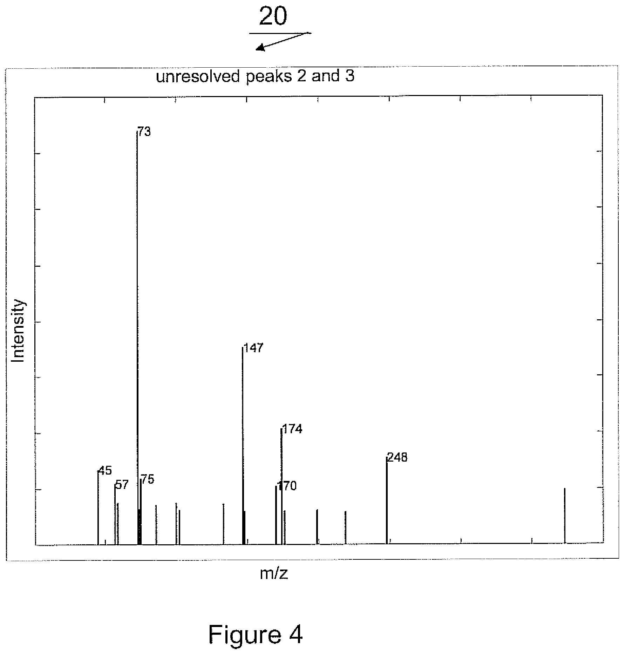

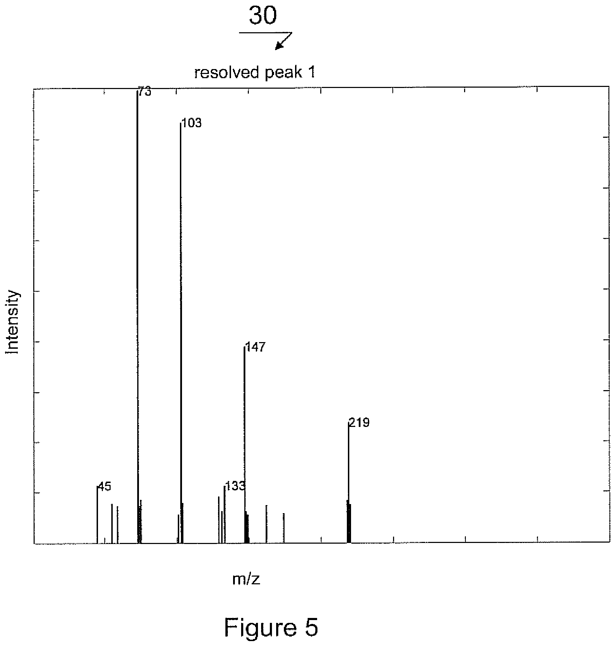

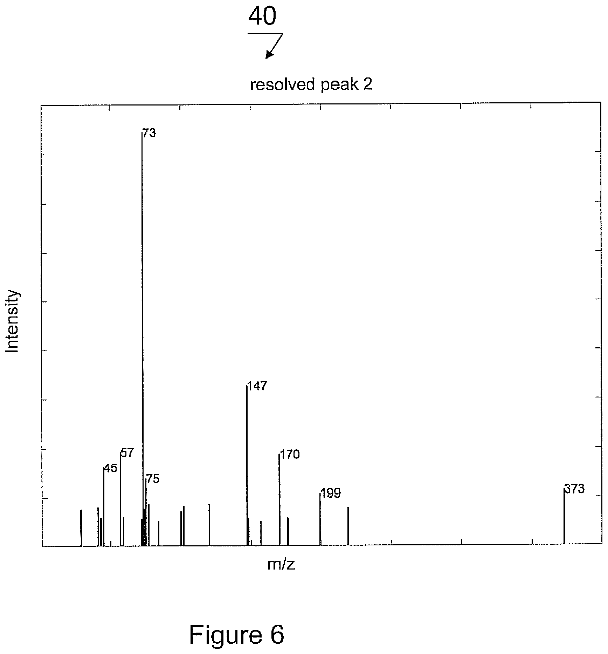

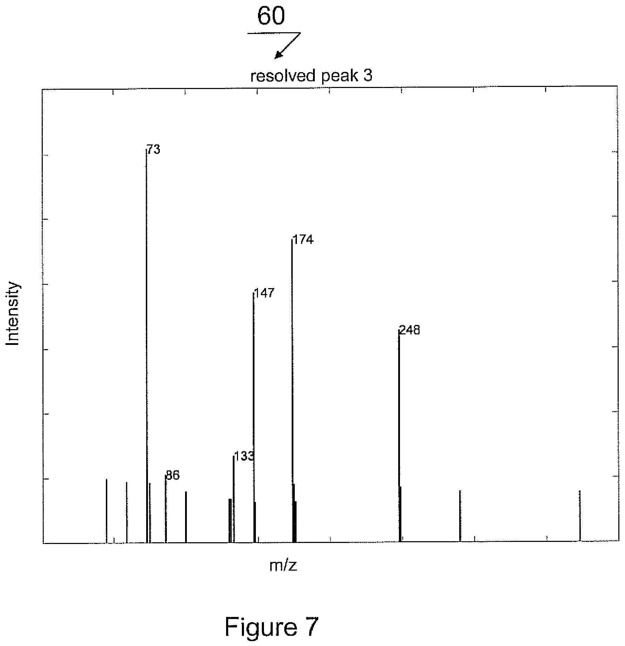

Figure 2 depicts in a schematic way an embodiment of a chromatogram 10 comprising three convoluted peaks 1, 2, and 3 which are presented as a function of retention time. The chromatogram 10 may be generated by means of gas chromatography which generally involves a vaporised sample which is injected onto a head portion of a chromatographic column. The sample is usually transported through the column by a flow on inert, gaseous mobile phase. The column may comprise a liquid stationary phase which is adsorbed onto a surface of an inert solid. The detector used for generating chromatographic data may refer to a so-called concentration-dependent detector, which is known per se. It will be appreciated that the methodology of the gas chromatography is known per se. It will be further appreciated that the chromatogram 10 may comprise more or less individual peaks, wherein a degree of mutual overlap between such peaks may also be different from sample to sample. Figure 3 depicts in a schematic way an embodiment of resolved peaks 1', 2', 3' substantially constituting the chromatogram 10 of Figure 2. Figure 4 depicts in a schematic way convoluted spectra 20 for peaks 2 and 3 of Figure 2, wherein individual peaks constituting the convoluted spectra may be taken from a suitable database of characteristic peaks. Figure 5 depicts in a schematic way characteristic peaks 30 constituting the spectrum of peak 1 of Figure 2 and Figure 6 depicts in a schematic way characteristic peaks 40 constituting the spectrum of peak 2 of Figure 2. Figure 7 depicts in a schematic way characteristic peaks 60 constituting the spectrum of peak 3 of Figure 2. It will be appreciated that the characteristic peaks 30, 40 and 60 in accordance with the invention are taken from the a-priori compiled database of characteristic peaks which are most probably present in the sample under consideration. It is possible that such database is compiled in an empirical way, i.e. based on experience of a particular lab, or an experience with particular samples. It is also possible that the database is compiled using standard databases of chromatograms of chemical species, or based on an abstract of such standard database.

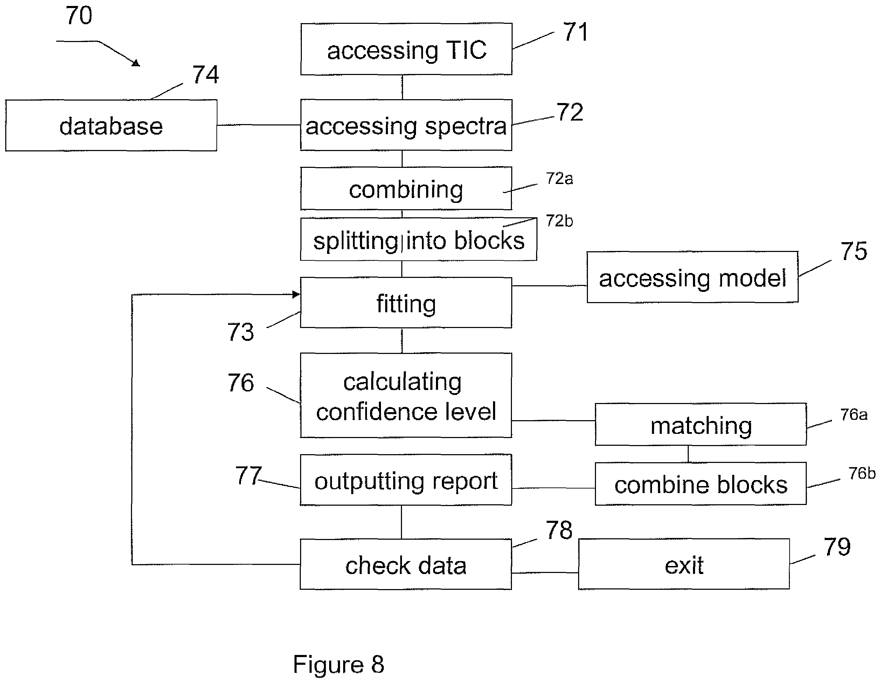

Figure 8 depicts in a schematic way an embodiment of a flow-chart of a computer program product according to the invention for enabling simultaneous deconvolution of peaks in multiple chromatographic spectra using database of characteristic peaks of compounds. The computer program product 70 may relate to an executable file comprising instructions for causing a processor of a suitable computer to carry out the steps of the method according to the invention. Accordingly, at step 71 the computer program product, embodiment of which is discussed herein below with reference to a flow-chart representation, may comprise an instruction for causing the processor to access a suitable chromatographic spectrum. It will be appreciated that suitable spectra may be collected online, may be downloaded from a suitable server or may be retrieved in any other suitable way. Such operation may be carried out by setting suitable input means into a data 'receive' mode. For example, a suitable chromatography spectrum may be accessed from a memory of a computer, or, alternatively from a remote site. For the latter local area network or internet facilities may be used. The spectra may be converted into a suitable format for processing, for example into NETCDF format.

After the chromatographic spectra data are accessed, the computer program according to the invention by means of instruction 72 causes the processor to access characteristic spectra of compounds expected to be found in the samples analyzed by means of chromatography from a database 74, wherein the characteristic spectra are supposed to be used for deconvoluting the multiple chromatographic spectra under consideration. In order to purposefully select the characteristic spectra, the computer program may access a data identifier (not shown), which may be stored in the file of the chromatographic spectrum for source identification purpose. For example, should the chromatographic spectrum correspond to a medical sample, the corresponding data may be stored in a pre-determined format, wherein identification of expected sample constituents is notified.

At step 72b the multiple spectra are split into blocks or data, preferably into overlapping blocks representing suitable strips of retention time windows. Preferably, retention times of blocks overlap 50% with a preceding and 50% with a following block of data. This is found to be advantageous for improving reliability of data extraction.

At step 73, the computer program comprises an instruction for the processor for carrying out a step of numerical fitting of the chromatographic spectrum for deconvoluting peaks. For example, the spectra may be aligned by auto cross correlation. Additionally or alternatively baseline from each individual trace may be removed. Next, a number of components per block may be estimated by rank analysis. Alternative least squares may be used for calculating both spectrum and chromatogram of a component using the reference database of expected components and their characteristic spectra. For this purpose at step 75 a suitable fitting model, of a multivariate statistical analysis, like equations of a regression analysis, may be accessed. After this stem peaks constituting the chromatographic spectrum accessed at step 71 are deconvoluted.

In order to quantify accuracy of the deconvolution, the computer program according to the invention comprises instruction 76 for causing the processor to calculate a confidence level related to accuracy of deconvolution, for example related to accuracy of identification of a sub-peak.

At step 76a during extraction reliability of each subsequent extracted component is checked by matching spectra against a database of known components. If identity is found then the program may proceed, if not the program may stop.

At step 76b information from current and following block of data may be combined. Peaks having most reliable area of the block may be extracted. Usually, a central area of the block represents the most reliable area.

At step 77 the computer program according to the invention may cause the processor to generate a report in a suitable form, for example, the report may relate to a table, a hard-copy print, or a file stored in a suitable format, for example in a HTML format. After the report is generated, the computer program may proceed to a further instruction 78 for checking output data, wherein peaks having confidence level lower than a pre-set threshold confidence level are suitably marked. Although in a preferred embodiment the computer program could proceed automatically to refitting at least the peaks with inferior confidence level, it is also possible that such peaks may be refitted manually. Finally, the computer program stops its operation by proceeding to the exit instruction.

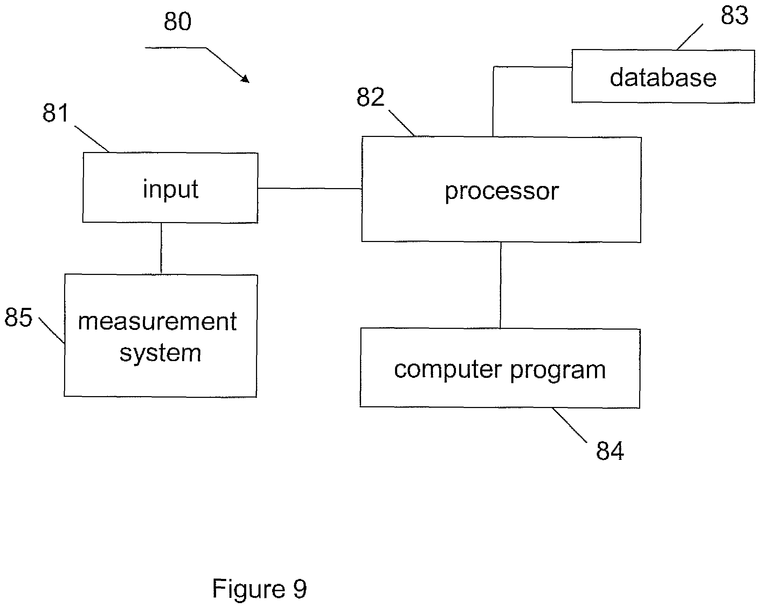

Figure 9 depicts in a schematic way an embodiment of a system according to the invention. A system 80 according to the invention comprises a processor 82, which, preferably, makes part of a suitable computer. The system 80 may further comprise a suitable database 83 of the characteristic peaks which may be assessed automatically by the system upon deconvoluting a chromatographic spectrum. The database may be stored in a memory unit, or may be provided on a suitable data carrier, like a CD-ROM, a flash memory disk or any other suitably readable medium. It is further possible that the database is stored remotely with respect to the processor and that processor establishes a data communication link with the database when a chromatographic spectrum is to be resolved.

In order to access data the system 80 comprises an input unit 81, which may be connectable to an output (not shown) of a suitable measurement system 82 arranged for generating chromatographic spectra. Preferably, such measurement system forms part of the system 80 according to the invention.

The system 80 according to the invention further comprises a computer program product 84, which is explained in detail with reference to Figure 8, said computer program product 84 being arranged to operate a processor 82 in accordance with the method of the invention. The system 80 may further comprise suitable display for providing a feed-back to a user, as well as other suitable periphery units, like printers, modems, or the like.

It will be appreciated that while specific embodiments of the invention have been described above, that the invention may be practiced otherwise than as described. In addition, isolated features discussed with reference to different figures may be combined.

## Claims

1. Method of simultaneous data processing for an automatic extraction of respective mass peaks in multiple chromatographic spectra of a chromatogram comprising convoluted peaks, comprising the steps of:
- accessing a database of characteristic mass peaks of known reference compounds;
- combining the multiple chromatographic spectra into a single block of data along a retention time scale;
- splitting the single block of data in smaller partly overlapping individual retention time blocks having a retention time window larger than an estimated peak width of an average single peak of the chromatogram;
- selecting multiple partly overlapping individual blocks of data in said multiple chromatographic spectra for deconvolution;
- deconvoluting the individual blocks of data into the respective mass peaks based on a plurality of characteristic mass peaks selected from said database using prior knowledge about expected constituents of the chromatographic spectrum, using multivariate statistical analysis, wherein extraction of mass peaks is terminated when a match is found between the extracted spectra and characteristic mass peaks of a reference compound.

2. Method according to claim 1, comprising the step of assigning a confidence level to the extracted peaks in the chromatogram.

3. Method according to claim 2, wherein in the confidence level a threshold level is set.

4. Method according to claim 2 or 3, further comprising a step of generating a report on respective peaks, wherein at least the peaks with a confidence level below the threshold level are marked.

5. Method according to any one of the preceding claims, the method further comprising the step of extracting residual mass peaks from the chromatographic spectrum based on a residual signal.

6. Method according to any one of the preceding claims, wherein the chromatographic spectrum is provided in an electronic file comprising data at least partially identifying expected constituents of the chromatographic spectrum, the method further comprising the step of using said data for the prior knowledge.

7. Computer program product comprising instructions for causing a processor to carry out the steps of the method as claimed in claims 1 -6.

8. System (80) for data processing for an automatic extraction of respective peaks in multiple chromatographic spectra of a chromatogram comprising convoluted peaks, comprising:
- an input (81) for accessing a database (83) of characteristic mass peaks of known reference compounds;
- a processor (82) for
- combining the multiple chromatographic spectra into a single block of data along a retention time scale;
- splitting the single block of data in smaller partly overlapping individual retention time blocks having a retention time window larger than an estimated peak width of an average single peak of the chromatogram;
- selecting multiple partly overlapping individual blocks of data in said multiple chromatographic spectra for deconvolution;
- deconvoluting the individual blocks of data into the respective mass peaks based on a plurality of characteristic mass peaks selected from said database using prior knowledge about expected constituents of the chromatographic spectrum, using multivariate statistical analysis, wherein extraction of mass peaks is terminated when a match is found between the extracted spectra and characteristic mass peaks of a reference compound.

9. System according to claim 8, further comprising the database (83) of characteristic peaks.

10. System according to claim 8 or 9 further comprising a gas chromatography unit.

## Patentansprüche

1. Verfahren zur gleichzeitigen Datenverarbeitung zum automatischen Extrahieren der jeweiligen Massenpeaks in mehreren chromatographischen Spektren eines Chromatogramms mit gefalteten Peaks, umfassend die Schritte:
- Zugriff auf eine Datenbank charakteristischer Massenpeaks bekannter Referenzverbindungen;
- Kombinieren der mehreren chromatographischen Spektren zu einem einzelnen Datenblock entlang einer Retentionszeitskala;
- Aufteilen des einzelnen Datenblocks in kleinere, teilweise überlappende einzelne Retentionszeitblöcke mit einem Retentionszeitfenster, das größer als eine geschätzte Peakbreite eines durchschnittlichen einzelnen Peaks des Chromatogramms ist;
- Auswählen mehrerer teilweise überlappender einzelner Datenblöcke in den mehreren chromatographischen Spektren zur Entfaltung;
- Entfalten der einzelnen Datenblöcke in die jeweiligen Massenpeaks basierend auf mehreren charakteristischen Massenpeaks, die aus der Datenbank ausgewählt wurden, unter Verwendung von Vorwissen über erwartete Bestandteile des chromatographischen Spektrums unter Verwendung einer multivariaten statistischen Analyse, wobei das Extrahieren von Massenpeaks beendet wird, wenn eine Übereinstimmung zwischen den extrahierten Spektren und den charakteristischen Massenpeaks einer Referenzverbindung vorliegt.

2. Verfahren nach Anspruch 1, umfassend den Schritt des Zuweisens eines Konfidenzniveaus den extrahierten Peaks in dem Chromatogramm.

3. Verfahren nach Anspruch 2, wobei in dem Konfidenzniveau ein Schwellenwert eingestellt ist.

4. Verfahren nach Anspruch 2 oder 3, ferner umfassend einen Schritt des Erzeugens eines Berichts über jeweilige Peaks, wobei wenigstens die Peaks mit einem Konfidenzniveau unter dem Schwellenwert markiert sind.

5. Verfahren nach einem der vorhergehenden Ansprüche, wobei das Verfahren ferner den Schritt des Extrahierens von Restmassenpeaks aus dem chromatographischen Spektrum basierend auf einem Restsignal umfasst.

6. Verfahren nach einem der vorhergehenden Ansprüche, wobei das chromatographische Spektrum in einer elektronischen Datei bereitgestellt wird, die Daten umfasst, die wenigstens teilweise erwartete Bestandteile des chromatographischen Spektrums identifizieren, wobei das Verfahren ferner den Schritt der Verwendung dieser Daten für das Vorwissen umfasst.

7. Computerprogrammprodukt, umfassend Anweisungen zum Bewirken, dass ein Prozessor die Schritte des Verfahrens nach den Ansprüchen 1 bis 6 ausführt.

8. System (80) zur Datenverarbeitung zum automatischen Extrahieren der jeweiligen Peaks in mehreren chromatographischen Spektren eines Chromatogramms, das gefaltete Peaks umfasst, umfassend:
- eine Eingabe: (81) zum Zugreifen auf eine Datenbank (83) charakteristischer Massenpeaks bekannter Referenzverbindungen;
- einen Prozessor (82) zum
- Kombinieren der mehreren chromatographischen Spektren zu einem einzelnen Datenblock entlang einer Retentionszeitskala;
- Aufteilen des einzelnen Datenblocks in kleinere, teilweise überlappende einzelne Retentionszeitblöcke mit einem Retentionszeitfenster, das größer als eine geschätzte Peakbreite eines durchschnittlichen einzelnen Peaks des Chromatogramms ist;
- Auswählen mehrerer teilweise überlappender einzelner Datenblöcke in den mehreren chromatographischen Spektren zur Entfaltung;
- Entfalten der einzelnen Datenblöcke in die jeweiligen Massenpeaks basierend auf mehreren charakteristischen Massenpeaks, die aus der Datenbank ausgewählt wurden, unter Verwendung von Vorwissen über erwartete Bestandteile des chromatographischen Spektrums unter Verwendung einer multivariaten statistischen Analyse, wobei das Extrahieren von Massenpeaks beendet wird, wenn eine Übereinstimmung zwischen den extrahierten Spektren und den charakteristischen Massenpeaks einer Referenzverbindung vorliegt.

9. System nach Anspruch 8, ferner umfassend die Datenbank (83) von charakteristischen Peaks.

10. System nach Anspruch 8 oder 9, ferner umfassend eine Gaschromatographieeinheit.

## Revendications

1. Procédé de traitement simultané de données pour une extraction automatique des pics de masse respectifs dans de multiples spectres chromatographiques d'un chromatogramme comprenant des pics convolués, comprenant les étapes consistant à :
- accéder à une base de données de pics de masse caractéristiques de composés de référence connus ;
- combiner les multiples spectres chromatographiques en un bloc unique de données le long d'une échelle de temps de rétention ;
- diviser le bloc unique de données en blocs de temps de rétention individuels plus petits se chevauchant partiellement ayant une fenêtre de temps de rétention plus grande qu'une largeur de pic estimée d'un pic unique moyen du chromatogramme ;
- sélectionner de multiples blocs de données individuels se chevauchant partiellement dans lesdits multiples spectres chromatographiques pour une déconvolution ;
- déconvoluer les blocs de données individuels en les pics de masse respectifs sur la base d'une pluralité de pics de masse caractéristiques sélectionnés dans ladite base de données en utilisant une connaissance antérieure concernant des constituants attendus du spectre chromatographique, en utilisant une analyse statistique multivariée, dans lequel l'extraction de pics de masse se termine lorsqu'une correspondance est trouvée entre les spectres extraits et des pics de masse caractéristiques d'un composé de référence.

2. Procédé selon la revendication 1, comprenant l'étape d'attribution d'un niveau de confiance aux pics extraits dans le chromatogramme.

3. Procédé selon la revendication 2, dans lequel, dans le niveau de confiance, un niveau de seuil est établi.

4. Procédé selon la revendication 2 ou 3, comprenant en outre une étape de génération d'un rapport sur des pics respectifs, dans lequel au moins les pics ayant un niveau de confiance inférieur au niveau de seuil sont marqués.

5. Procédé selon l'une quelconque des revendications précédentes, le procédé comprenant en outre l'étape d'extraction de pics de masse résiduels à partir du spectre chromatographique sur la base d'un signal résiduel.

6. Procédé selon l'une quelconque des revendications précédentes, dans lequel le spectre chromatographique est fourni dans un fichier électronique comprenant des données identifiant au moins partiellement des constituants attendus du spectre chromatographique, le procédé comprenant en outre l'étape consistant à utiliser lesdites données pour la connaissance antérieure.

7. Produit de programme informatique comprenant des instructions pour amener un processeur à effectuer les étapes du procédé selon les revendications 1 à 6.

8. Système (80) pour le traitement de données pour une extraction automatique de pics respectifs dans de multiples spectres chromatographiques d'un chromatogramme comprenant des pics convolués, comprenant :
- une entrée (81) pour accéder à une base de données (83) de pics de masse caractéristiques de composés de référence connus ;
- un processeur (82) pour
- combiner les multiples spectres chromatographiques en un bloc unique de données le long d'une échelle de temps de rétention ;
- diviser le bloc unique de données en blocs de temps de rétention individuels plus petits se chevauchant partiellement ayant une fenêtre de temps de rétention plus grande qu'une largeur de pic estimée d'un pic unique moyen du chromatogramme ;
- sélectionner de multiples blocs de données individuels se chevauchant partiellement dans lesdits multiples spectres chromatographiques pour une déconvolution ;
- déconvoluer les blocs de données individuels en les pics de masse respectifs sur la base d'une pluralité de pics de masse caractéristiques sélectionnés dans ladite base de données en utilisant une connaissance antérieure concernant des constituants attendus du spectre chromatographique, en utilisant une analyse statistique multivariée, dans lequel l'extraction de pics de masse se termine lorsqu'une correspondance est trouvée entre les spectres extraits et des pics de masse caractéristiques d'un composé de référence.

9. Système selon la revendication 8, comprenant en outre la base de données (83) de pics caractéristiques.

10. Système selon la revendication 8 ou 9 comprenant en outre une unité de chromatographie en phase gazeuse.
